(19) Europäisches Patentamt / European Patent Office / Office européen des brevets

(11) **EP 1 184 028 B2**

(12) **NOUVEAU FASCICULE DE BREVET EUROPEEN**
Après la procédure d'opposition

(45) Date de publication et mention de la décision concernant l'opposition:
**02.12.2009 Bulletin 2009/49**

(51) Int Cl.:
***A61Q 1/06*** *(2006.01)*     ***A61K 8/37*** *(2006.01)*

(45) Mention de la délivrance du brevet:
**09.03.2005 Bulletin 2005/10**

(21) Numéro de dépôt: **01402099.4**

(22) Date de dépôt: **02.08.2001**

(54) **Composition cosmétique sans transfert comprenant un composé non volatil siliconé, une huile hydrocarbonée non volatile et une phase particulaire inerte**

Abriebfeste Kosmetikzusammensetzung enthaltend ein nichtflüchtiges Silicon, ein nichtflüchtiges Kohlenwasserstofföl und eine inerte Teilchenphase

Non-transfer cosmetic composition comprising a non-volatile silicone, a non-volatile hydrocarbon oil and an inert particulate phase

(84) Etats contractants désignés:
**AT BE CH CY DE DK ES FI FR GB GR IE IT LI LU MC NL PT SE TR**

(30) Priorité: **30.08.2000 FR 0011081**

(43) Date de publication de la demande:
**06.03.2002 Bulletin 2002/10**

(73) Titulaire: **L'ORÉAL**
**75008 Paris (FR)**

(72) Inventeur: **Arnaud, Pascal**
**94240 l'Hay les Roses (FR)**

(74) Mandataire: **Chantraine, Sylvie Hélène**
**L'ORÉAL - D.I.P.I.**
**25-29, Quai Aulagnier**
**92600 Asnieres (FR)**

(56) Documents cités:
| | |
|---|---|
| EP-A- 0 797 976 | EP-A1- 1 008 339 |
| EP-A1- 1 023 890 | EP-A2- 0 437 216 |
| EP-A2- 1 055 424 | WO-A1-00/00163 |
| WO-A1-00/66076 | WO-A1-99/27903 |
| DE-A1- 4 303 983 | US- - 4 853 222 |
| US- - 4 894 222 | US- - 5 013 763 |
| US- - 5 039 518 | US- - 5 498 406 |
| US- - 5 738 841 | US- - 5 871 756 |
| US-A- 5 034 216 | US-A- 5 648 066 |
| US-A- 5 690 918 | |

- 'CTFA-online excerpt for octyl palmitate (ethylhexyl palmitate)'
- ' Structure of ethylhexylpamitate'
- ' CTFA-online excerpt for octyl methoxycinnamate'
- ' CTFA-online excerpt for glyceryl tricaprate (tricaprin)'
- 'CTFA-online excerpt for isostearyl isostearate'
- ' CTFA-online excerpt for cetyl rininoleate'
- ' CTFA-online excerpt for glyceryl tricaprylate (tricaprylin)'
- 'Dow Corning Material Safety Data Sheet for DC 556'
- ' Dow Corning Product Information Sheet for DC556'
- 'Data Sheet in relation to Ceraphyl 368'
- 'Fluid Characteristic Chart, including castoir oil'
- 'CTFA-online excerpt for octyl dodecanol'
- 'CTFA-online excerpt for isobutyl palmitate'
- 'CTFA-online excerpt for isopropylisostearate'
- 'CTFA-online excerpt for ethylhexyl stearate (octylstearate)'
- 'CTFA-online excerpt for glyceryl caprylate'
- 'CTFA-online excerpt for neopentyl glycol diocatanoate'
- 'CTFA-online excerpt for isostearyl palmytate'
- 'INCI dictionay and Handbook 8th', 2000 page 1135
- 'CTFA-online excerpt for "Suspending Agents-Non surfactant"'
- 'Cosmetic and Toiletry Formulation, 2d Edition'
- 'Präsentation"DUB VCI 10 Velvet Ester" (Stéarinerie Dubois)'
- ' Kosmetikjahrbuch 1997 Rohstoff -Lexikon', 1997, VERLAG FÜR CHEMISCHE INDUSTRIE, AUSBURG article H.ZIOLKOWSKY GMBH, page 479

    **(Cont. page suivante)**

- 'Degussa, Care Specialties: ABIL-Wax-Cosmetic oils/waxes for skin care products'
- 'Tego Cosmetics; W/O-Cremes und W/O-Lotionen, A' Août 1992,
- 'ABIL-Wax 9800,ABIL-Wax 9801,ABIL-Wax 2434,ABIL-Wax 2440,Fiche du fabricant'
- 'Dow Corning: Think Dow Corning Silicones for personal care A unique marketing proposition'
- 'Dow Corning 1401,Fiche du fabricant,' Décembre 1989,
- 'Dow Corning Q2-1403, Fiche du fabricant,' Juillet 1989,
- 'CTFA,édition de 1997,vol.2,' pages 1033 - 1034
- 'Fiche sécurité du produit GE Bayer silicones 001752/03,Baysilone Fluid PD7'
- 'CTFA,édition de 1997,vol.2,' pages 431 - 432

**Description**

La présente invention a trait à une composition contenant un composé non volatil siliconé et une huile hydrocarbonée non volatile, compatibles entre eux, destinée en particulier au domaine cosmétique. Plus spécialement, l'invention se rapporte à une composition sans transfert et brillante pour le soin et/ou le maquillage de la peau aussi bien du visage que du corps humain, des lèvres, des paupières inférieures ou supérieures ou encore des phanères comme les cils, les sourcils, les ongles et les cheveux.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eye liners, les mascaras, les compositions de protection solaire, de coloration ou de bronzage artificiel de la peau ou encore de maquillage du corps ou des cheveux.

Les produits de maquillage ou de soin de la peau ou des lèvres des êtres humains comme les fonds de teint ou les rouges à lèvres contiennent généralement des phases grasses telles que des cires et des huiles, des pigments et/ou charges et éventuellement des additifs comme des actifs cosmétiques ou dermatologiques. Elles peuvent aussi contenir des produits dits "pâteux", de consistance souple, permettant d'obtenir des pâtes, colorées ou non, à appliquer au pinceau.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, comme notamment un verre, une tasse, une cigarette, un vêtement ou de la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

De plus, ces compositions ont tendance à migrer, c'est-à-dire à se propager à l'intérieur des rides et des ridules de la peau qui entourent les lèvres et les yeux, entraînant un effet inesthétique.

La société Procter & Gambie a envisagé dans sa demande de brevet WO-A-96/40044 des compositions de rouge à lèvres présentant des propriétés de sans transfert, contenant une huile volatile et une huile non volatile du type perfluoropolyéther, incompatibles. Dans cette demande, il est, en outre, décrit l'amélioration de la brillance, grâce à la dispersion préalable d'une phase huileuse dans une matrice, et la capacité de cette phase huileuse à ségréguer lors de l'application du produit sur le support et à migrer à la surface du film ainsi déposé.

Ce système nécessite cependant une bonne dispersion de la phase huileuse dans la matrice et peut engendrer des problèmes de stabilité du produit, liés à la nécessaire mauvaise compatibilité de la phase huileuse avec la matrice.

Il est connu par ailleurs que l'amélioration des propriétés de brillance nécessite une bonne dispersion des particules solides dans la composition en particulier des pigments.

Le brevet US-A-5 945 092 de Revlon décrit ainsi l'utilisation de tensioactifs siliconés associés avec des huiles volatiles.

Malgré leur efficacité, ces tensioactifs présentent l'inconvénient d'être potentiellement irritant notamment pour la muqueuse labiale lorsque leur pourcentage dans la composition est important (typiquement supérieur à 3 %), et ceci d'autant plus que le taux d'huile volatile est élevé (supérieur à 30 % typiquement).

Il est donc particulièrement intéressant de trouver un autre moyen d'améliorer la brillance des compositions sans transfert sans avoir les inconvénients cités précédemment.

De plus, les compositions contenant des huiles volatiles, bien que présentant des propriétés de "sans transfert" améliorées ont l'inconvénient de laisser sur les lèvres, après évaporation de ces huiles volatiles, un film qui devient très vite inconfortable au cours du temps (sensation de dessèchement, de tiraillement et d'inconfort), écartant un certain nombre de femmes de ce type de rouge à lèvres. De plus, le dépôt obtenu est mat. Or les consommateurs sont toujours à la recherche d'un produit brillant, confortable à porter tout au long de la journée, qui ne migre pas ou peu dans les plis de la peau entourant les lèvres ou les yeux et qui ne transfère pas ou pratiquement pas.

Il subsiste donc le besoin d'une composition ne présentant pas les inconvénients ci-dessus et ayant notamment de bonnes propriétés de "sans transfert" et de non-migration, même lors d'une pression ou d'un frottement prononcé, tout en conférant au dépôt un aspect plus ou moins brillant, adapté au désir de la consommatrice, ne desséchant pas et ne tiraillant pas la peau ou les lèvres sur lesquelles elle est appliquée, aussi bien lors de l'application qu'au cours du temps.

La demanderesse a constaté, de façon tout à fait surprenante, que l'utilisation d'un composé non volatil siliconé, d'une phase particulaire inerte et d'une huile hydrocarbonée non volatile de faible masse moléculaire, compatible avec le composé non volatil siliconé, dans une composition physiologiquement acceptable et plus spécialement cosmétique, permettait d'obtenir un dépôt brillant, de très bonne tenue, ne transférant peu ou pas du tout, ne migrant pas, résistant à l'eau, tout en étant très agréable à l'application et à porter tout au long de la journée. Le dépôt est souple et onctueux.

Par "compatible", on entend que le composé siliconé soit soluble ou dispersible dans l'huile hydrocarbonée non volatile, à chaud et à température ambiante et forme, à l'oeil nu, une phase homogène. De préférence, le composé siliconé est soluble dans l'huile hydrocarbonée.

La présente invention a donc pour objet une composition anhybre de soin ou de maquillage des matières kératiniques, comprenant

- au moins une huile non volatile hydrocarbonée présentant une masse molaire allant de 230 à 420g/Mol, présente à un taux massique compris entre 5 et 60%,
- au moins un composé siliconé non volatil, compatible avec l'huile non volatile hydrocarbonée, présente à un taux massique compris entre 0,5 à 90%,
- un agent dispersant comprenant au moins un composé non volatil hydrocarboné compatible avec lesdites huiles non volatiles hydrocarbonées et incompatible avec le composé non volatil siliconé, ledit agent dispersant étant choisi parmi le malate de diisostéaryle, les acides poly(hydroxy-12) stéariques, et leurs mélanges, et ledit agent dispersant étant présent en une teneur massique de 2 à 40 %,
- une phase particulaire inerte,

et contenant moins de 10% en poids d'huiles volatiles.

De façon avantageuse, l'huile hydrocarbonée non volatile, appelée aussi solvant non volatil, de faible masse moléculaire, présente une masse moléculaire allant de 240 à 350 g/Mol, de préférence de 240 à 300 g/Mol et mieux de 240 à 280 g/Mol. Avantageusement, la composition est exempte de silicone volatile et encore mieux exempte de tout solvant volatil.

Par "huile", on entend tout milieu non aqueux liquide à température ambiante (25°C) et pression atmosphérique (760mm de Hg).

Par milieu "non-volatil", on entend tout milieu susceptible de rester sur la peau ou les lèvres pendant plusieurs heures. Un milieu non-volatil a en particulier une pression de vapeur, à température ambiante et pression atmosphérique, non nulle, inférieure à 0,02 mm de Hg (2,66 Pa).

Par "volatil", on entend un milieu susceptible de s'évaporer de la peau ou des lèvres, en moins d'une heure. Un milieu volatil est notamment choisi parmi les milieux ayant une pression de vapeur, à température ambiante et pression atmosphérique allant de 0,02 mm à 300mm de Hg (2,66 Pa à 40 000 Pa) et mieux allant de 0,1 à 90 mm de Hg (13 Pa à 12 000 Pa). En outre, les silicones volatiles présentent généralement une viscosité inférieure à 5 cSt à température ambiante et pression atmosphérique.

La composition peut contenir une ou plusieurs huiles non volatiles hydrocarbonées et un ou plusieurs composés non volatils siliconés.

Par "phase particulaire inerte", on entend toute charge solide à température ambiante et pression atmosphérique, utilisée seule ou en association, ne réagissant pas chimiquement avec les différents ingrédients de la composition et qui sont insolubles dans ces ingrédients, même lorsque ces ingrédients sont portés à une température supérieure à la température ambiante (température de fusion par exemple de ces ingrédients).

Cette composition est en particulier une composition cosmétique ou dermatologique. Elle contient donc des ingrédients compatibles avec les matières kératiniques, à savoir la peau, les lèvres, les fibres kératiniques et les ongles. Elle peut se présenter sous forme de gel anhydre. Elle peut se présenter, en outre, sous forme plus ou moins fluide, de pâte ou de solide non déformable ou rigide, éventuellement coulé en stick ou en coupelle. De préférence, elle se présente sous forme fluide ou de stick anhydre. Par "fluide", on entend une composition s'écoulant sous son propre poids, à l'inverse d'un solide.

Selon l'invention, le composé non volatil siliconé et l'huile non volatile hydrocarbonée sont compatibles entre eux. Le dépôt obtenu sur la peau ou les lèvres est homogène et souple. Il ne laisse pratiquement pas de traces sur un support venant au contact du dépôt et ne migre pas dans les rides et ridules autour des lèvres notamment.

Avantageusement, la composition comprend un agent dispersant de particules solides qui contient au moins un composé hydrocarboné non volatil compatible avec l'huile hydrocarbonée non volatile et incompatible avec le composé non volatil siliconé, l'agent dispersant ayant des paramètres de solubilité tels que $16,40 \ (J/cm^3)^{1/2} \le \delta_D \le 19,00 \ (J/cm^3)^{1/2}$ et $2,00 \ (J/cm^3)^{1/2} \le \delta_a \le 9,08 \ (J/cm^3)^{1/2}$. Le composé hydrocarboné non volatil est incompatible avec le composé non volatil siliconé, c'est à dire non soluble ou non dispersable dans le composé non volatil siliconé.

L'agent dispersant permet la dispersion de toutes les particules solides, à température ambiante et pression atmosphérique, présentes dans la composition, telles que les charges, les pigments et les nacres.

La composition selon l'invention comprend donc un ou plusieurs composés hydrocarbonés non volatils physiologiquement acceptables, servant de dispersant des particules solides.

Avantageusement, la composition contient au moins un ingrédient choisi parmi les actifs cosmétiques ou dermatologiques, les matières colorantes et leurs mélanges. L'incompatibilité du composé non volatil siliconé et du dispersant hydrocarboné non volatil permet notamment de contribuer à la limitation, voire suppression du transfert de la composition et en particulier du transfert des actifs et/ou des matières colorantes. Ainsi, il est possible de maintenir ces actifs et/ou matières colorantes là où ils ont été déposés, tout en conférant à la composition des propriétés de confort du fait du remplacement, par rapport à l'art antérieur, des solvants volatils par des huiles non volatiles de faible masse moléculaire. L'huile non volatile hydrocarbonée de faible masse moléculaire sert à compatibiliser (solubiliser ou disperser et mieux, solubiliser) le composé siliconé non volatil et l'agent dispersant. En l'absence de l'huile non volatile de faible masse moléculaire, le composé siliconé et l'agent dispersant forment deux phases non miscibles, à chaud et à température ambiante.

Un autre objet de l'invention est l'utilisation cosmétique, dans une composition cosmétique ou pour la fabrication d'une composition à application topique, contenant un agent dispersant comprenant au moins un composé non volatil hydrocarboné présentant des paramétres de solubilité tels que $16,40 \ (J/cm^3)^{1/2} \le \delta_D \le 19,00 (J/cm^3)^{1/2}$ et $2,00 \ (J/cm^3)^{1/2} \le \delta_a \le 9,08 \ (J/cm^3)^{1/2}$, de l'association d' au moins une huile non volatile hydrocarbonée présentant une masse molaire allant de 230 à 420g/Mol, présente à un taux massique compris entre 5 et 60%, d'au moins un composé siliconé non volatil, compatible avec l'huile non volatile hydrocarbonée, présente à un taux massique compris entre 0,5 à 90%, et d'une phase particulaire inerte, ladite composition contenant moins de 10% en poids d'huiles volatiles, pour diminuer, voire supprimer, le transfert d'un film de composition déposé sur la peau et/ou les lèvres d'être humain vers un support mis en contact avec le film et/ou pour conserver la brillance du film et/ou pour apporter à ce film du confort et/ou pour augmenter la tenue du film dans le temps et/ou pour réduire la migration du film.

L'invention a encore pour objet un procédé cosmétique de soin ou de maquillage des lèvres, des phanères ou de la peau, consistant à appliquer sur respectivement les lèvres, les phanères ou la peau une composition cosmétique telle définie précédemment.

On a de plus constaté que la composition selon l'invention, présente des qualités d'étalement et d'adhésion sur la peau et les lèvres, particulièrement intéressantes, ainsi qu'un toucher onctueux et agréable. Cette composition a, en outre, l'avantage de se démaquiller facilement notamment avec un lait démaquillant classique. Ceci est tout à fait remarquable puisque les compositions de l'art antérieur à propriétés "sans transfert" élevées sont très difficiles à démaquiller. En général, elles sont vendues avec un produit démaquillant spécifique, ce qui introduit une contrainte supplémentaire pour l'utilisatrice.

Les composés non volatils siliconés de l'invention doivent être solubles ou dispersibles dans la ou les huiles non volatiles hydrocarbonées et notamment dans les esters non volatils de masse moléculaire de 230 à 420 g/Mol. Ils sont de préférence choisis parmi les composés liquides à température ambiante et de manière encore plus préférentielle, ils ont une viscosité comprise dans la gamme allant de 5 à 1 000 000 cSt à 25°C et mieux de 10 à 500 000 cSt, de préférence de 10 à 5 000 cSt.

A titre d'exemples de composés siliconés, on peut citer les polydiméthylsiloxanes, les phényltriméthicones, les polyalkylméthylsiloxanes, les résines de silicones telles celles décrites dans les documents JP-A-62-61911, JP-A-61-65809 et EP-A-602905, les silicones fluorées et leurs mélanges.

En particulier, ces composés siliconés sont choisis parmi les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates; les silicones fluorées comportant un groupement fluoré pendant ou en bout de chaîne siliconée ayant de 1 à 12 atomes de carbone dont tout ou partie des hydrogènes est substitué par des atomes de fluor ; les résines de silicone; les gommes de silicone dont les diméthiconols ; et leurs mélanges.

Le taux massique de composé siliconé dans la composition finale est compris dans la gamme allant de 0,5 à 90 % et de préférence de 5 à 60 % et encore mieux de préférence de 10 à 50 %.

Le composé non volatil siliconé est de préférence en proportion égale ou supérieure à celle du composé hydrocarboné non volatil, servant de dispersant ; autrement dit, le rapport R défini par :

$$R = \frac{\% \ massique \ de \ composé \ non \ volatil \ siliconé}{\% \ massique \ d'agent \ dispersant}$$

est de préférence égal ou supérieur à 1.

Les composés hydrocarbonés non volatils de l'invention, jouant le rôle de dispersant des particules solides doivent aussi être compatibles avec les huiles non volatiles hydrocarbonées de faible masse moléculaire mais par contre ils ne doivent pas être compatibles avec les composés siliconés précédemment décrits.

Ces agents dispersants sont fluides à température ambiante et notamment liquides.

De préférence, les composés hydrocarbonés non volatils servant de dispersant selon l'invention sont tels que leurs paramètres de solubilité de HANSEN, $\delta_D$, $\delta_p$, $\delta_h$ sont tels que :

$16,40 \ (J/cm^3)^{1/2} \le \delta_D \le 19,00 \ (J/cm^3)^{1/2}$ et de préférence $16,70 \ (J/cm^3)^{1/2} \le \delta_D \le 18,50 \ (J/cm^3)^{1/2}$;

2,00 (J/cm$^3$)$^{½}$ ≤δ$_a$ ≤ 9,08 (J/cm$^3$)$^{½}$ et de préférence 4,00 (J/cm$^3$)$^{½}$ ≤ δ$_a$ ≤ 9,08 (J/cm$^3$)$^{½}$ et encore de préférence 5,00 (J/cm$^3$)$^{½}$ ≤δ$_a$≤ 6,80 (J/cm$^3$)$^{½}$

sachant que δ$_a$ = (δ$_p$$^2$ + δ$_h$2)$^{½}$.

La définition des paramètres de solubilité selon HANSEN est bien connue de l'homme du métier, et notamment décrite dans l'article de C. M. HANSEN : "The three dimensional solubility parameters" J. Paint Technol. 39,105 (1967). Ces paramètres sont aussi décrits dans le document JP-A-08-109121 de KAO et le document de D.W. Van KREVELEN "Properties of polymers" (1990), p. 190°

Selon cet espace de HANSEN :

- δ$_D$ caractérise les forces de dispersion de LONDON issues de la formation de dipôles induits lors des chocs moléculaires ;
- δ$_P$ caractérise les forces d'interactions de DEBYE entre dipôles permanents ;
- δ$_h$ caractérise les forces d'interactions spécifiques (type liaisons hydrogène, acide/base, donneur/accepteur, etc.) ;

Les paramètres δ$_D$, δ$_P$ et δ$_h$ sont généralement exprimés en (J/cm$^3$)$^{½}$. Ils sont déterminés à température ambiante (25°C) et en particulier selon la méthode de calcul indiquée dans le document brevet de KAO ci-dessus.

Dans ce cas, les paramètres de solubilité du mélange sont déterminés à partir de ceux des dispersants fluides pris séparément, selon les relations suivantes :

$$\delta_{Dmel} = \sum_i xi\, \delta_{Di} \quad ;$$

$$\delta_{pmel} = \sum_i xi\, \delta_{pi}$$

et

$$\delta_{hmel} = \sum_i xi\, \delta_{hi}$$

où xi représente la fraction volumique du dispersant fluide hydrocarboné non volatil (i) dans le mélange.

De préférence, la masse moléculaire des dispersants fluides hydrocarbonés est supérieure à 600 g/Mol et de préférence supérieure à 700 g/Mol.

A titre de dispersant hydrocarboné non volatil utilisable dans l'invention, on utilise le malate de diisostéaryle, des acides poly(hydroxy-12) stéariques tels que le Solsperse 21 000 vendu par la société Zénéca ou l'Arlacel P100 vendu par la société Uniqema et leurs mélanges. De préférence, on utilise les acides poly (hydroxy-12) stéariques

Le taux massique de dispersant, dans la composition finale, est compris dans la gamme allant de 2 à 40 % et de préférence de 2,5 à 20 % et mieux de 3 à 10 %.

Comme solvant ou huile non volatile hydrocarboné de faible masse moléculaire utilisable dans l'invention, on peut citer les esters sous forme monoester, diester et de façon générale polyester de masse moléculaire allant de 230 à 420 g/Mol.

Les esters peuvent être linéaires ou ramifiés, saturés ou non. De préférence, ils sont sous forme ramifiée et saturée. Ces esters sont de préférence des esters d'acide en C$_2$ à C$_{18}$ et notamment d'alcool en C$_2$ à C$_{20}$ ou de polyol en C$_2$ à C$_8$ ou de leurs mélanges. Comme huile solvant non volatile utilisable dans l'invention, on peut citer les esters de l'acide néopentanoïque comme l'isodecyl neopentanoate (242,4), l'isotridecyl neopentanoate (270,44), l'isostearyl neopentanoate (354,62), l'octyldocecyl neopentanoate (382,67) ; les esters de l'acide isononanoïque comme l'isononyl isononanoate (284,48), l'octyl isononanoate (270,44), l'isodecyl isononanoate (298,51), l'isotridecyl isononanoate (340,59), l'isostearyl isononanoate (410,73), mais aussi les esters de l'alcool isopropylique, tels que l'isopropyl myristate (270,46), l'isopropyl palmitate (298,51), l'isopropyl stearate ou isostearate, le cetyl octanoate (368,64), le tridecyl octanoate (326,55), le PEG-4diheptanoate (418,51) et l'éthyl 2-hexyl palmitate (368,64), le C$_{12}$-C$_{15}$ alkyl benzoate (309,04), le neopentyl glycol diheptanoate (328,49), le propyleneglycol diethyl 2-hexanoate. Ces esters sont cités en noms CTFA (International Cosmetic Ingredient Dictionary, 5$^{ème}$ édition et suivantes). On peut aussi citer les alcanes comme l'isoéïcosane (282,55). Les chiffres entre parenthèses correspondent à leur masse moléculaire exprimée en g/Mol.

De préférence, on utilise les esters de l'acide néopentanoïque ou isononanoïque.

Le ou les solvants non volatils hydrocarbonés de faible masse moléculaire selon l'invention, représente de préférence de 10 à 60 % et mieux de 15 à 50 %, et encore mieux de 15 à 30 % du poids total de la composition.

La composition peut, en outre, contenir au moins un corps gras additionnel différent du composé non volatil siliconé, du solvant ou huile non volatil hydrocarboné et du composé dispersant hydrocarboné non volatil, choisi parmi les cires, les gommes, les corps gras pâteux à température ambiante, les huiles et leurs mélanges, d'origine minérale, animale, végétale ou de synthèse.

Les corps gras huileux additionnels de la composition peuvent être une huile cosmétiquement ou dermatologiquement acceptable, et de façon générale physiologiquement acceptable, notamment choisie parmi les huiles d'origine minérale, animale, végétale ou synthétique, volatiles ou non volatiles.

Comme huiles additionnelles utilisables dans la composition selon l'invention, on peut citer notamment :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame ou de colza, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le Parleam ;
- les esters et les éthers de synthèse notamment d'acides gras comme les huiles de formule $R_3COOR_4$ dans laquelle $R_3$ représente le reste d'un acide gras supérieur comportant de 1 à 40 atomes de carbone et $R_4$ représente une chaîne hydrocarbonée contenant de 1 à 40 atomes de carbone avec $R_3 + R_4 \geq 27$ comme par exemple l'huile de Purcellin, le stéarate d'octyl 2-dodécyle, l'érucate d'octyl 2-dodécyte, l'isostéarate d'isostéaryle ; les esters hydroxylés comme l'hydroxy stéarate d'octyl dodécyle, le citrate de tri-isocétyle, des heptanoates, octanoates, décanoates d'alcools gras ; des esters de polyol comme le di-isostéarate de propylène glycol ; et les esters du pentaérythritol comme le tétra-isostéarate de penta-érythrytyle ;
- des alcools gras ayant de 12 à 26 atomes de carbone comme l'octyl dodécanol, le 2-butyl octanol, le 2-hexyl décanol, le 2-undécylpenta décanol, l'alcool oléique ;
- les huiles fluorées non volatiles, partiellement hydrocarbonées et/ou siliconées, comme le méthoxynonafluorobutane ;
- leurs mélanges.

La ou les huiles additionnelles non volatiles de la composition peuvent représenter de 0,1 % à 90 % du poids total de la composition, de préférence de 5 à 60 % et mieux de 10 à 50 %.

De préférence, la composition de l'invention contient peu ou pas d'huiles volatiles de préférence moins de 5 % du poids total de la composition et mieux moins de 2 % et avantageusement est exempte d'huile volatile.

La composition contient, en outre, une phase particulaire inerte qui contient au moins une charge inerte absorbante ou non, c'est-à-dire une ou plusieurs charges inertes en particulier absorbant les huiles. De préférence, ces charges ont un diamètre apparent allant de 0,01 à 150 $\mu$m et mieux de 0,5 à 150 $\mu$m. Un diamètre apparent correspond au diamètre du cercle dans lequel s'inscrit la particule élémentaire selon sa plus petite dimension (épaisseur pour des lamelles).

Les charges peuvent être minérales ou organiques, lamellaires, sphériques ou oblongues. On peut citer le talc, le mica, la silice, le kaolin, les poudres de polyamide comme le Nylon® (Orgasol® de chez Atochem), de paly-$\beta$-alanine et de polyéthylène, les poudres de polytétrafluoroéthylène (Téflon®), la lauroyl-lysine, l'amidon, le nitrure de bore, les microsphères creuses polymériques telles que celles de chlorure de polyvinylidène/acrytonitrile comme l'Expancel® (Nobel Industrie), les copolymères d'acide acrylique (Palytrap® de Dow Corning) et les microbilles de résine de silicone (Tospearl® de Toshiba, par exemple), le carbonate de calcium précipité, le carbonate et l'hydrocarbonate de magnésium, l'hydroxyapatite, les microsphères de silice creuses (Silica Beads® de Maprecos), les microcapsules de verre ou de céramique et leurs mélanges.

La phase particulaire inerte peut représenter de 0,1 à 30 % du poids total de la composition et mieux de 2 à 25 % et encore mieux de 10 à 20 %.

Les quantités respectives de composés siliconés non volatils, d'huile hydrocarbonée de faible masse moléculaire et de phase particulaire inerte sont choisies en quantité suffisante pour conférer à la composition des propriétés de non-transfert, de brillance et de confort.

La composition de l'invention peut comprendre avantageusement une ou plusieurs matières colorantes contenant au moins (un ou plusieurs) composés pulvérulents et/ou un ou plusieurs colorants liposolubles ou hydrosolubles, par exemple à raison de 0 à 70% du poids total de la composition et notamment de 0,01 à 70%. Le ou les composés pulvérulents peuvent être choisis parmi les pigments, les nacres, habituellement utilisés dans les compositions cosmétiques ou dermatologiques et leurs mélanges. Avantageusement, les composés pulvérulents colorants représentent jusqu'à 50 % du poids total de la composition, par exemple de 0,01 à 50 % en poids et mieux de 1 à 40 %.

Les pigments peuvent être blancs ou colorés, minéraux et/ou organiques, interférentiels ou non, insolubles dans la phase grasse liquide, destinées à colorer et/ou opacifier la composition. On peut citer, parmi les pigments minéraux, le dioxyde de titane, éventuellement traité en surface, les oxydes de zirconium ou de cérium, ainsi que les oxydes de zinc, de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0 à 20 % et notamment 0,01 à 20 % du poids de la composition et mieux de 0,1 à 6 %. Les colorants hydrosolubles sont par exemple le jus de betterave, le bleu de méthylène et peuvent représenter jusqu'à 6 % du poids total de la composition.

La composition de l'invention peut, en outre, contenir un ou plusieurs actifs cosmétiques ou dermatologiques tels que ceux classiquement utilisés.

Comme actifs cosmétiques ou dermatologiques, utilisables dans la composition de l'invention, on peut citer les hydratants, vitamines, acides gras essentiels, sphingolipides, filtres solaires, agents apaisants (Bisabolol, par exemple). Ces actifs sont utilisés en quantité habituelle pour l'homme du métier et notamment à des concentrations de 0 à 20 % et notamment de 0,001 à 20 % du poids total de la composition, et mieux de 0,1 à 5 %. La composition selon l'invention peut, de plus, comprendre, selon le type d'application envisagée, les constituants classiquement utilisés dans les domaines considérés, qui sont présents en une quantité appropriée à la forme galénique souhaitée.

Parmi les cires solides à température ambiante, susceptibles d'être présentes dans la composition selon l'invention, on peut citer les cires hydrocarbonées telles que la cire d'abeillés éventuellement modifiée, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch et les alcools gras en $C_{20}$-$C_{50}$. On peut également utiliser des cires de silicone, parmi lesquelles on peut citer les alkyl, alcoxy et/ou esters de polyméthylsiloxane et leurs mélanges.

Les cires peuvent être présentes à raison de 0-50% (par exemple 0,01 à 50 %) en poids dans la composition et mieux de 5 à 20 %, en vue de ne pas trop diminuer la brillance de la composition et du film déposé sur les lèvres et/ou la peau.

Comme corps gras pâteux, on peut citer des corps gras ayant un point de fusion allant de 25 à 45 °C et/ou une viscosité à 40 °C allant de 0,1 à 40 Pa.s mesurée au Contraves TV équipé d'un mobile MS-r3 ou Ms-r4 tournant à 60 Hz. A titre d'exemple de corps gras pâteux, on peut citer les PDMS ayant des chaînes pendantes du type alkyle ou alcoxy ayant de 8 à 24 atomes de carbone comme le stearyl diméthicone ; les esters d'alcool gras ou d'acide gras comme les esters du cholestérol, le polylaurate de vinyle, le propionate d'arachidyle ; le copolymère PVP/Eicosène ; les lanolines et leurs dérivés comme les lanolines acétylées ou les lanolines oxypropylénées ; et leurs mélanges.

La nature et la quantité des cires, des corps gras pâteux et des gommes sont fonction des propriétés mécaniques et de textures recherchées.

La composition peut comprendre, en outre, tout additif usuellement utilisé dans de telles compositions, tel que des épaississants (hectorite modifiée par chlorure de distéaryl di-méthyl ammonium par exemple connue sous le nom de Bentone®), des antioxydants, des parfums, des conservateurs, des tensioactifs, des polymères liposolubles comme les polyalkylènes, notamment le polybutène, les polyacrylates et les polymères siliconés compatibles avec la phase grasse ainsi que les dérivés de polyvinylpyrrolidone. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge ou encore dans une bouillotte. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide. Elles peuvent alors constituer des fonds de teint ou des rouges à lèvres, des brillants à lèvres (gloss en terminologie anglo-saxonne), des produits solaires ou de coloration de la peau.

Les compositions de l'invention sont anhydres. Elles peuvent alors se présenter notamment sous forme de gel huileux, de liquide huileux, de pâte ou de stick ou encore sous forme de dispersion vésiculaire contenant des lipides ioniques et/ou non ioniques. Ces formes galéniques sont préparées selon les méthodes usuelles des domaines considérés.

Ces compositions à application topique peuvent constituer notamment une composition cosmétique ou dermatologique, de protection, de traitement ou de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

L'invention est illustrée plus en détail dans les exemples suivants: Les pourcentages sont des pourcentages massiques. Les noms de certains ingrédients sont donnés en nom CTFA.

**Exemple 1 : Rouge à lèvres en stick**

### *Phase A*

- Acide poly(hydroxy -12) stéarique 3,14 vendu sous la référence Solsperse 21000 par la Société Zeneca
- Bis diglycéryl poly acyl adipate-2 3,13
- Lanoline 2,09
- Propionate d'Arachidyle 6,46
- Néopentanoate d'octyldodécyle 7,84
- Antioxydant 0,06

### *Phase B*

- Isononanoate d'isononyle 20,68
- Bentone 38V vendue et/ou fabriquée par la société SASI 1,32

### *Phase C*

- Ozokérite 1,8
- Cire de Polyéthylène ($M_n$*=500) 6,0
- Cire de Polyéthylène ($M_n$*=400) 3,0

* $M_n$ signifie la masse moléculaire moyenne en nombre.

### *Phase D*

- Dioxyde de titane 2,79
- DC Red N° 7 0,06
- Oxyde de fer noir 0,34
- Oxyde de fer rouge 0,84
- Lauroyl lysine 5,00
- Kaolin 10,43

### *Phase E*

- Phenyltrimethicone (1000 cSt) 15,60
- Phenyltrimethicone (20 cSt) 7,62

### Phase F

- Bisabolol     0,40

### Phase G

- Nacres     1,40          100,00% massique

**Mode opératoire**

La phase A est préparée en pesant successivement les constituants et en les mélangeant sous agitation à 80-90 °C. La phase B est préparée en dispersant la Bentone dans l'huile hydrocarbonée. On mélange ensuite les phases A et B à 80-90 °C.

La phase particulaire D est broyée dans une fraction des phases A+B à l'aide d'un broyeur tri-cylindre.

On ajoute à ce mélange la phase cireuse C et on chauffe l'ensemble à 100°C jusqu'à dissolution complète des cires. On ajoute ensuite les silicones de la phase E puis la phase F et enfin les nacres de la phase G, à 100°C.

On peut ensuite couler le mélange final obtenu à 100°C dans des moules pour obtenir des sticks.

Ce rouge à lèvres en stick est satiné à brillant, confortable à porter au cours du temps, non gras, non collant et présentant de bonnes propriétés de non-transfert.

**Exemples 2 à 6 : Rouge à lèvres en stick**

| Phase A | n°2 (invention) | n°3 (comparatif) | n°4 (comparatif) | n°5 (comparatif) | n°6 (comparatif) |
|---|---|---|---|---|---|
| - Acide poly(hydroxy-12) stéarique | 3,00 | 3,00 | 3,00 | - | - |
| - Huile de Ricin | - | - | - | - | 3,00 |
| - Bis iglycéryl polyacyladipate-2 | 3,00 | 3,00 | 3,00 | 3,00 | 3,00 |
| - Lanoline | 2,00 | 2,00 | 2,00 | 2,00 | 2,00 |
| - Propionate d'arachidyle | 6,18 | 6,18 | 6,18 | 6,18 | 6,18 |
| - Néopentanoate d'octyldodécyle | 7,50 | - | 7,50 | 7,50 | 7,50 |
| - Polyisobutène hydrogéné ($M_n$ =450,89) vendu sous la référence Parleam par Nippon Oil Fats | - | 7,50 | - | 3,00 | - |
| - Antioxydant | 0,06 | 0,06 | 0,06 | 0,06 | 0,06 |
| **Phase B** | | | | | |
| - Isononanoate d'isononyle | 20,68 | - | 20,68 | 20,68 | 20,68 |
| - Polyisobutène hydrogéné ($M_n$ = 450,89) vendu sous la référence Parleam par Nippon Oil Fats | - | 20,68 | - | - | - |
| - Bentone 38V | 1,32 | 1,32 | 1,32 | 1,32 | 1,32 |
| **Phase C** | | | | | |
| - Ozokérite | 2,00 | 2,00 | 2,00 | 2,00 | 2,00 |

(suite)

| | | | | | |
|---|---|---|---|---|---|
| ***Phase C*** | | | | | |
| - Cire de Polyéthylène ($M_n$= 500) | 6,67 | 6,67 | 6,67 | 6,67 | 6,67 |
| - Cire de Polyéthylène ($M_n$= 400) | 3,33 | 3,33 | 3,33 | 3,33 | 3,33 |
| ***Phase D*** | | | | | |
| - Dioxyde de titane | 0,33 | 0,33 | 0,33 | 0,33 | 0,33 |
| - DC Red 27 Al Lake - DC Red 7 | 0,43 | 0,43 | 0,43 | 0,43 | 0,43 |
| | 0,57 | 0,57 | 0,57 | 0,57 | 0,57 |
| - FD et C Yellow 6 Al Lake | 0,17 | 0,17 | 0,17 | 0,17 | 0,17 |
| - Lauroyl lysine | 5,00 | 5,00 | 5,00 | 5,00 | 5,00 |
| - Kaolin | 13,00 | 13,00 | 13,00 | 13,00 | 13,00 |
| ***Phase E*** | | | | | |
| - Phenyltrimethicone (1000 cSt) | 15,60 | 15,60 | - | 15,60 | 15,60 |
| - Polybutène vendu sous la référence Dynapak H 100 par la Société Pakhoed Products | - | - | 14,63 | - | - |
| - Phenyltrimethicone (20 cSt) | 7,62 | 7,62 | - | 7,62 | 7,62 |
| - Polyisobutène hydrogéné ($M_n$ = 450,89) vendu sous la référence Parleam par Nippon Oil Fats | - | - | 8,59 | - | - |
| ***Phase F*** | | | | | |
| - Bisabolol | 0,40 | 0,40 | 0,40 | 0,40 | 0,40 |
| ***Phase G*** | | | | | |
| - Nacres | 1,14 | 1,14 | 1,14 | 1,14 | 1,14 |
| | -------- | -------- | -------- | -------- | -------- |
| | 100,00 % massique | | | | |

### Mode opératoire

Les exemples 2 à 6 ont été réalisés suivant le même mode opératoire que celui décrit pour l'exemple 1.
Les propriétés sensorielles des exemples comparatifs 3, 4, 5 et 6 ont été comparées avec celles de l'exemple 2 selon l'invention.

### *Comparaison Exemple 2 et Exemple 3*

Les esters de faibles masses moléculaires, que sont l'isononyl isononanoate et l'octyldodécyl néopentanoate de l'exemple 2 ont été substitués dans l'exemple 3 par le polyisobutène hydrogéné dont la masse moléculaire est de 450,89 g/Mol.
Les propriétés non-transfert de la composition de l'exemple 2 ont été jugées supérieures à celles de la composition de l'exemple 3. Elles ont été évaluées 15 minutes après application du rouge à lèvres, par embrassement d'une feuille de papier (test du bisou).
Les esters de faibles masses moléculaires sont donc favorables au non-transfert. Le rouge à lèvres de l'exemple 2 a été jugé satiné à brillant et confortable.

### *Comparaison Exemple 2 et Exemple 4*

Les silicones que sont les phenyltrimethicones (20 et 1000 cSt) de l'exemple 2 ont été substituées par du polybutène et le polyisobutène hydrogéné dans l'exemple 4. Les proportions de ces alcanes ont été choisies de manière à obtenir une viscosité proche de celle du mélange de silicones.

Les propriétés non-transfert de la composition de l'exemple 2 ont été jugées supérieures à celles de la composition de l'exemple 4. Par contre, cette dernière a conduit à un film trop huileux.

Les silicones non volatiles sont donc favorables au non-transfert, sans apport de gras sur les lèvres.

*Comparaison Exemple 2 et Exemple 5*

Le dispersant (Solperse 21 000) de l'exemple 2 a été substitué dans l'exemple 5 par du polyisobuténe hydrogéné dont la masse moléculaire (450,89 g/Mol) est supérieure à celles des huiles hydrocarbonées de faible masse moléculaire de l'invention et les paramètres de solubilité de Hansen $\delta_D$ et $\delta_a$ sont respectivement de 15,48 et 0 $(J/cm^3)^{1/2}$.

La composition de l'exemple 5 conduit à un film sur les lèvres moins brillant que celui de la composition de l'exemple 2 pour un transfert similaire.

Le dispersant de l'invention est donc favorable à la brillance du film pour des propriétés non-transfert comparables.

*Comparaison Exemple 2 et Exemple 6*

Le dispersant Solperse 21 000 de l'exemple 2 a été substitué dans l'exemple 6 par de l'Huile de Ricin dont le paramètre de solubilité $\delta_a$ vaut 9,09 $J/cm^3)^{1/2}$.

L'exemple 6 conduit à un film sur les lèvres moins brillant que celui de la composition de l'exemple 2 pour un transfert similaire.

Le dispersant de l'invention est donc favorable à la brillance du film pour des propriétés non-transfert comparables.

Les exemples 2 à 6 montrent clairement que l'association huile hydrocarbonée non volatile / composé siliconé non volatil / charges contenant éventuellement un agent dispersant de particules solides confère à la composition des propriétés de sans transfert améliorées par rapport à l'art antérieur, sans nuire aux propriétés de brillance et tout en conférant du confort.

## Exemple 7 : Rouge à Lèvres en stick (exemple comparatif)

### *Phase A*

- Acide poly(hydroxy-12) stéarique       3,00
- Bis diglycéryl polyacyladipate-2       3,00
- Lanoline       2,00
- Propionate d'arachidyle       6,18
- Antioxydant       0,06

### *Phase B*

- Ozokérite       2,00
- Cire de polyéthylène ($M_n$ = 500)       6,67
- Cire de polyéthylène ($M_n$ = 400)       3,33

### *Phase C*

- Dioxyde de Titane       0,33
- DC Red 27 Al Lake       0,43
- DC Red 7       0,57
- FD et C Yellow 6 Al Lake       0,17
- Lauroyl Lysine       5,00

- Bentone 38V      1,32

*Phase D*

- Kaolin      13,00

*Phase E*

- Phenyltrimethicone (1000 cSt)      15,60
- Phenyltrimethicone (20 cSt)      7,62

*Phase F*

- Bisabolol      0,40

*Phase G*

- Nacres      1,14

*Phase H*

- Cyclotetradimethylsiloxane (D4)      28,18          100,00 % massique

**Mode opératoire**

La phase A est préparée en pesant successivement les constituants et en les mélangeant sous agitation à 80 - 90°C.

La phase particulaire colorée C est broyée dans la phase A à l'aide d'un broyeur tri-cylindre.

La phase E est préparée en pesant successivement les constituants et en les mélangeant sous agitation.

La phase particulaire inerte D est broyée dans la phase E à l'aide d'un broyeur tri-cylindre.

On réunit ensuite les mélangés A+C et D+E et on ajoute la phase cireuse B. On chauffe l'ensemble à 100°C jusqu'à dissolution complète des cires.

On ajoute la phase F puis la phase G et enfin la phase H, cette dernière étant introduite à 90°C.

On coule ensuite le mélange final dans des moules adéquats pour obtenir des sticks.

*Comparaison Exemple 2 et Exemple 5*

Les esters de faibles masses moléculaires que sont l'isononyle isononanoate et octyldodecyl néopentanoate de l'exemple 2 ont été substitués dans l'exemple 7 par une silicone volatile (D4).

La composition de l'exemple 7 a été jugée très rêche à l'application et a conduit à un film très mat et très collant sur les lèvres, à l'inverse de la composition de l'exemple 2. Les huiles hydrocarbonées de faible masse moléculaire sont donc favorables à la brillance et au confort.

**Revendications**

1. Composition anhydre de soin ou de maquillage des matières kératiniques, comprenant

- au moins une huile non volatile hydrocarbonée présentant une masse molaire allant de 230 à 420g/Mol, présente à un taux massique compris entre 5 et 60%, - au moins un composé siliconé non volatil, compatible avec l'huile non volatile hydrocarbonée, présent à un taux massique allant de 0,5 à 90%,

un agent dispersant comprenant au moins un composé non volatil hydrocarboné compatible avec lesdites huiles non volatiles hydrocarbonées et incompatible avec le composé non volatil siliconé, ledit agent dispersant étant choisi parmi le malate de diisostéaryle, les acides poly(hydroxy-12) stéariques et leurs mélanges,

et ledit agent dispersant étant présent en une teneur massique allant de 2 à 40 %,

- une phase particulaire inerte,

et contenant moins de 10% en poids d'huiles volatiles.

**2.** Composition selon la revendication 1, **caractérisée en ce que** l'huile non volatile hydrocarbonée présente une masse moléculaire allant de 240 à 350 g/Mol, de préférence de 240 à 300 g/Mol et mieux de 240 à 280 g/Mol.

**3.** Composition selon la revendication 1 ou 2, **caractérisée en ce que** l'huile non volatile hydrocarbonée est un ester.

**4.** Composition selon l'une des revendications précédentes, **caractérisée en ce que** l'huile non volatile hydrocarbonée est un ester d'acide en $C_2$ à $C_{18}$.

**5.** Composition selon l'une des revendications précédentes, **caractérisée en ce que** l'huile non volatile hydrocarbonée est choisie parmi les esters d'alcool en $C_2$ à $C_{20}$, les esters de polyol en $C_2$ à $C_8$ et leurs mélanges.

**6.** Composition selon l'une de revendications précédentes, **caractérisée en ce que** l'huile non volatile hydrocarbonée est un ester d'acide ramifié.

**7.** Composition selon l'une des revendications précédentes, **caractérisée en ce que** l'huile non volatile hydrocarbonée est choisie parmi les esters de l'acide néopentanoïque, les esters de l'acide isononanoïque et leurs mélanges.

**8.** Composition selon l'une des revendications précédentes, **caractérisée en ce que** l'huile non volatile hydrocarbonée est choisie parmi l'isodecyl neopentanoate, l'isotridecyl neopentanoate, l'isostearyl neopentanoate, l'octyldocecyl neopentanoate, l'isononyl isononanoate, l'octyl isononanoate, l'isodecyl isononanoate, l'isotridecyl isononanoate, l'isostéaryl isononanoate et leurs mélanges.

**9.** Composition selon l'une des revendications précédentes, **caractérisée en ce que** l'agent dispersant présente des paramètres de solubilité tels que $16,40 \ (J/cm^3)^{1/2} \leq \delta_D \leq 19,00 \ (J/cm^3)^{1/2}$ et $2,00 \ (J/cm^3)^{1/2} \leq \delta_a \leq 9,08 \ (J/cm^3)^{1/2}$.

**10.** Composition selon l'une des revendications précédentes, **caractérisée en ce que** l'agent dispersant présente des paramètres de solubilité tels que $16,70 \ (J/cm^3)^{1/2} \leq \delta_D \leq 18,50 \ (J/cm^3)^{1/2}$.

**11.** Composition selon l'une des revendications 1 à 10, **caractérisée en ce que** l'agent dispersant présente des paramètres de solubilité tels que $4,00 \ (J/cm^3)^{1/2} \leq \delta_a \leq 9,08 \ (J/cm^3)^{1/2}$ et mieux $5,00 \ (J/cm^3)^{1/2} \leq \delta_a \leq 6,80 \ (J/cm^3)^{1/2}$.

**12.** Composition selon l'une des revendications 1 à 11, **caractérisée en ce que** l'agent dispersant présente une masse molaire supérieure à 600g/Mol, de préférence supérieure à 700 g/Mol.

**13.** Composition selon l'une des revendications 1 à 12, **caractérisée en ce que** l'agent dispersant est présent en une teneur massique allant de 2,5 à 20 % et mieux de 3 à 10 %.

**14.** Composition selon l'une des revendications précédentes, **caractérisée en ce que** le composé non volatil siliconé est choisi parmi les composés liquides à température ambiante.

**15.** Composition selon l'une des revendications précédentes, dans laquelle le composé non volatil siliconé présente une viscosité choisie dans la gamme allant de 5 à 1 000 000 cSt et de préférence allant de 10 à 500 000 cSt et mieux de 10 à 5 000 cSt.

**16.** Composition selon l'une des revendications précédentes, dans laquelle le composé non volatil siliconé est choisi parmi les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ;

les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les silicones fluorées comportant un groupement fluoré pendant ou en bout de chaîne siliconée ayant de 1 à 12 atomes de carbone dont tout ou partie des hydrogènes est substitué par des atomes de fluor ; les résines de silicone ; les gommes de silicone ; les diméthiconols ; et leurs mélanges.

17. Composition selon l'une des revendications précédentes, **caractérisée en ce que** le composé non volatil siliconé représente un taux massique de 5 à 60 %, et mieux de 10 à 50%.

18. Composition selon l'une des revendications précédentes, dans laquelle l'huile non volatile hydrocarbonée représente un taux massique de 10 à 60 % et mieux de 15 à 50 %.

19. Composition selon l'une des revendications précédentes, dans laquelle au moins un ingrédient choisi parmi les actifs cosmétiques, les actifs dermatologiques, les matières colorantes et leurs mélanges est prévu.

20. Composition selon l'une des revendications 1 à 19 dans laquelle le rapport massique de composé siliconé volatil par rapport à l'agent dispersant est égal ou supérieur à 1.

21. Composition selon l'une des revendications 1 à 20, **caractérisée en ce qu'**elle comprend, en outre, au moins un corps gras différent du composé siliconé non volatil, de l'huile non volatile hydrocarbonée et de l'agent dispersant choisi parmi les cires, les gommes, les corps gras pâteux à température ambiante, les huiles et leurs mélanges.

22. Composition selon l'une des revendications 19 à 21, dans laquelle les matières colorantes comprennent au moins un composé pulvérulent colorant choisi parmi les pigments, les nacres et leurs mélanges.

23. Composition selon la revendication 22, **caractérisée en ce que** le composé pulvérulent colorant représente jusqu'à 50 % du poids total de la composition.

24. Composition selon l'une des revendications précédentes, **caractérisée en ce que** la phase particulaire contient au moins une charge inerte absorbante ou non.

25. Composition selon la revendication 24, **caractérisée en ce que** la charge inerte est choisie parmi les charges sphériques, lamellaires, oblongues et leurs mélanges.

26. Composition selon la revendication 24 ou 25, **caractérisée en ce que** la charge inerte est choisie parmi le talc, le mica, la silice, le kaolin, les poudres de polyamide, de poly-$\beta$-alanine et de polyéthylène, les poudres de polytétrafluoroéthylène, la lauroyl-lysine, l'amidon, le nitrure de bore, les microsphères creuses polymériques, les copolymères d'acide acrylique, les microbilles de résine de silicone, le carbonate de calcium précipité, le carbonate et l'hydro-carbonate de magnésium, l'hydroxyapatite, les microsphères de silice creuses, les microcapsules de verre ou de céramique et leurs mélanges.

27. Composition selon l'une des revendications précédentes, **caractérisée en ce que** la phase particulaire inerte représente de 0,1 à 30 % du poids total de la composition, de préférence de 2 à 25 % et mieux de 10 à 20 %.

28. Composition selon l'une des revendications précédentes, **caractérisée en ce qu'**elle contient moins de 5% en poids d'huiles volatiles.

29. Composition selon l'une des revendications précédentes, **caractérisée en ce qu'**elle est exempte d'huile volatile.

30. Composition selon l'une des revendications précédentes, se présentant sous forme d'un stick ou bâton, sous la forme d'une pâte souple, sous forme de coupelle, de gel huileux, de liquide huileux, de dispersion vésiculaire contenant des lipides ioniques et/ou non ioniques, d'émulsion eau-dans-huile ou huile-dans-eau.

31. Composition de maquillage en particulier de la peau ou des lèvres, **caractérisée en ce qu'**elle est conforme à l'une des revendications précédentes.

32. Composition selon l'une des revendications précédentes, se présentant sous forme d'un fond de teint, d'un fard à joues ou à paupières, d'un rouge à lèvres, d'une base ou baume de soin pour les lèvres, d'un produit anti-cernes, d'un eye liner, d'un mascara.

33. Procédé de soin cosmétique ou de maquillage des lèvres ou de la peau, consistant à appliquer sur respectivement les lèvres ou la peau une composition cosmétique telle définie aux revendications 1 à 32.

34. Utilisation cosmétique dans une composition cosmétique ou à application topique contenant un agent dispersant comprenant au moins un composé non volatil hydrocarboné présentant des paramètres de solubilité tels que 16,40 $(J/cm^3)^{1/2} \leq \delta_D \leq 19,00$ $(J/cm^3)^{1/2}$ et 2,00 $(J/cm^3)^{1/2} \leq \delta_a \leq 9,08$ $(J/cm^3)^{1/2}$ et moins de 10% en poids du poids total de la composition d'huiles volatiles, de l'association

- d'au moins une huile non volatile hydrocarbonée présentant une masse molaire allant de 230 à 420g/Mol, présente à un taux massique compris entre 5 et 60%,
- d'au moins un composé siliconé non volatil, compatible avec l'huile non volatile hydrocarbonée présente à un taux massique compris entre 0,5% et 90%, et
- d'une phase particulaire inerte,

pour diminuer, voire supprimer, le transfert d'un film de composition déposé sur la peau et/ou les lèvres d'être humain vers un support mis en contact avec le film et/ou pour conserver la brillance et/ou pour apporter à ce film du confort et/ou pour réduire sa migration.

35. Utilisation selon la revendication 34, **caractérisée en ce que** la composition est exempte d'huile volatile.

**Claims**

1. Anhydrous care or make-up composition for keratin materials, comprising

- at least one non-volatile hydrocarbon-based oil with a molar mass ranging from 230 to 420 g/Mol, which is present in a mass fraction of between 5% and 60%,
- at least one non-volatile silicone compound which is compatible with the non-volatile hydrocarbon-based oil, which is present in a mass fraction of between 0.5% and 90%,
- a dispersant comprising at least one non-volatile hydrocarbon-based compound which is compatible with the said non-volatile hydrocarbon-based oils and incompatible with the non-volatile silicone compound, the said dispersant being chosen from diisostearyl malate, poly(12-hydroxystearic acids), and mixtures thereof, and the said dispersant being present in a mass fraction of between 2% and 40%,
- an inert particulate phase,

and containing less than 10% by weight of volatile oils.

2. Composition according to Claim 1, **characterized in that** the non-volatile hydrocarbon-based oil has a molecular mass ranging from 240 to 350 g/Mol, preferably from 240 to 300 g/Mol and better still from 240 to 280 g/Mol.

3. Composition according to Claim 1 or 2, **characterized in that** the non-volatile hydrocarbon-based oil is an ester.

4. Composition according to one of the preceding claims, **characterized in that** the non-volatile hydrocarbon-based oil is an ester of a $C_2$ to $C_{18}$ acid.

5. Composition according to one of the preceding claims, **characterized in that** the non-volatile hydrocarbon-based oil is chosen from esters of $C_2$ to $C_{20}$ alcohols and esters of $C_2$ to $C_8$ polyols, and mixtures thereof.

6. Composition according to one of the preceding claims, **characterized in that** the non-volatile hydrocarbon-based oil is a branched acid ester.

7. Composition according to one of the preceding claims, **characterized in that** the non-volatile hydrocarbon-based oil is chosen from neopentanoic acid esters and isononanoic acid esters, and mixtures thereof.

8. Composition according to one of the preceding claims, **characterized in that** the non-volatile hydrocarbon-based oil is chosen from isodecyl neopentanoate, isotridecyl neopentanoate, isostearyl neopentanoate, octyldodecyl ne-

EP 1 184 028 B2

opentanoate, isononyl isononanoate, octyl isononanoate, isodecyl isononanoate, isotridecyl isononanoate and isostearyl isononanoate, and mixtures thereof.

9. Composition according to one of the preceding claims, **characterized in that** the dispersant has solubility parameters such that 16.40 $(J/cm^3)^{1/2} \leq \delta_D \leq 19.00$ $(J/cm^3)^{1/2}$ and 2.00 $(J/cm^3)^{1/2} \leq \delta_a \leq 9.08$ $(J/cm^3)^{1/2}$.

10. Composition according to one of the preceding claims, **characterized in that** the dispersant has solubility parameters such that 16.70 $(J/cm^3)^{1/2} \leq \delta_D \leq 18.50$ $(J/cm^3)^{1/2}$.

11. Composition according to one of Claims 1 to 10, **characterized in that** the dispersant has solubility parameters such that 4.00 $(J/cm^3)^{1/2} \leq \delta_a \leq 9.08$ $(J/cm^3)^{1/2}$ and better still 5.00 $(J/cm^3)^{1/2} \leq \delta_a \leq 6.80$ $(J/cm^3)^{1/2}$.

12. Composition according to one of Claims 1 to 11, **characterized in that** the dispersant has a molar mass of greater than 600 g/Mol and preferably greater than 700 g/Mol.

13. Composition according to one of Claims 1 to 12, **characterized in that** the dispersant is present in a mass fraction ranging from 2.5% to 20% and better still from 3% to 10%.

14. Composition according to one of the preceding claims, **characterized in that** the non-volatile silicone compound is chosen from compounds that are liquid at room temperature.

15. Composition according to one of the preceding claims, in which the non-volatile silicone compound has a viscosity chosen within the range from 5 to 1 000 000 cSt, preferably ranging from 10 to 500 000 cSt and better still from 10 to 5000 cSt.

16. Composition according to one of the preceding claims, in which the non-volatile silicone compound is chosen from non-volatile polydimethylsiloxanes (PDMSs); polydimethylsiloxanes comprising alkyl, alkoxy or phenyl groups that are pendent or at the end of a silicone chain, these groups containing from 2 to 24 carbon atoms; phenyl trimethicones, phenyl dimethicones, phenyl trimethylsiloxydiphenylsiloxanes, diphenyl dimethicones, diphenyl methyldiphenyltrisiloxanes and 2-phenylethyl trimethylsiloxypilicates; fluorosilicones comprising a fluoro group which is pendent or at the end of a silicone chain and containing from 1 to 12 carbon atoms, all or some of the hydrogen atoms of which are replaced with fluorine atoms; silicone resins; silicone gums; dimethiconols; and mixtures thereof.

17. Composition according to one of the preceding claims, **characterized in that** the non-volatile silicone compound represents a mass fraction of from 5% to 60% and better still from 10% to 50%.

18. Composition according to one of the preceding claims, in which the non-volatile hydrocarbon-based oil represents a mass fraction of from 10% to 60% and better still from 15% to 50%.

19. Composition according to one of the preceding claims, in which at least one ingredient chosen from cosmetic active agents, dermatological active agents and dyestuffs, and mixtures thereof, is provided.

20. Composition according to one of Claims 1 to 19, in which the ratio by mass of volatile silicone compound relative to the dispersant is greater than or equal to 1.

21. Composition according to one of Claims 1 to 20, **characterized in that** it also comprises at least one fatty substance other than the non-volatile silicone compound, the non-volatile hydrocarbon-based oil and the dispersant, chosen from waxes, gums, fatty substances that are pasty at room temperature, and oils, and mixtures thereof.

22. Composition according to one of Claims 19 to 21, in which the dyestuffs comprise at least one pulverulent dye compound chosen from pigments and nacres, and mixtures thereof.

23. Composition according to Claim 22, **characterized in that** the pulverulent dye compound represents up to 50% of the total weight of the composition.

24. Composition according to one of the preceding claims, **characterized in that** the particulate phase contains at least one absorbent or non-absorbent inert filler.

17

**25.** Composition according to Claim 24, **characterized in that** the inert filler is chosen from spherical, lamellar and oblong fillers, and mixtures thereof.

**26.** Composition according to Claim 24 or 25, **characterized in that** the inert filler is chosen from talc, mica, silica, kaolin, polyamide powders, poly-β-alanine powder and polyethylene powder, polytetrafluoroethylene powders, lauroyllysine, starch, boron nitride, hollow polymer microspheres, acrylic acid copolymers, silicone resin microbeads, precipitated calcium carbonate, magnesium carbonate and hydrocarbonate, hydroxyapatite, hollow silica microspheres and glass or ceramic microcapsules, and mixtures thereof.

**27.** Composition according to one of the preceding claims, **characterized in that** the inert particulate phase represents from 0.1% to 30% of the total weight of the composition, preferably from 2% to 25% and better still from 10% to 20%.

**28.** Composition according to one of the preceding claims, **characterized in that** it contains less than 5% by weight of volatile oils.

**29.** Composition according to one of the preceding claims, **characterized in that** it is free of volatile oil.

**30.** Composition according to one of the preceding claims, which is in the form of a stick or tube, in the form of a soft paste, in the form of a dish, an oily gel, an oily liquid, a vesicular dispersion containing ionic and/or nonionic lipids, or a water-in-oil or oil-in-water emulsion.

**31.** Make-up composition, in particular for the skin or the lips, **characterized in that** it is in accordance with one of the preceding claims.

**32.** Composition according to one of the preceding claims, which is in the form of a foundation, a blusher, an eyeshadow, a lipstick, a care base or care balm for the lips, a concealer product, an eyeliner or a mascara.

**33.** Cosmetic care process or make-up process for the lips or the skin, which consists in applying a cosmetic composition as defined in Claims 1 to 32 to the lips or the skin, respectively.

**34.** Cosmetic use, in a cosmetic composition or in a composition for topical application containing a dispersant comprising at least one non-volatile hydrocarbon-based compound with solubility parameters such that $16.40\ (J/cm^3)^{1/2} \leq \delta_D \leq 19.00\ (J/cm^3)^{1/2}$ and $2.00\ (J/cm^3)^{1/2} \leq \delta_a \leq 9.08\ (J/cm^3)^{1/2}$ and less than 10% by weight, relative to the total weight of the composition, of volatile oils, of a combination

- of at least one non-volatile hydrocarbon-based oil with a molar mass ranging from 230 to 420g/Mol, which is present in a mass fraction of between 5% and 60%,
- of at least one non-volatile silicone compound which is compatible with the non-volatile hydrocarbon-based oil, which is present in a mass fraction of between 0.5% and 90%, and
- of an inert particulate phase, for reducing or even preventing altogether the transfer of a film of composition deposited on the skin and/or the lips of a human being to a support placed in contact with the film and/or for preserving its gloss and/or for making this film comfortable to wear and/or for reducing its migration.

**35.** Use according to Claim 34, **characterized in that** the composition is free of volatile oil.

**Patentansprüche**

**1.** Wasserfreie Zusammensetzung zur Pflege oder zum Schminken von Keratinsubstanzen, die enthält:

- mindestens ein nicht flüchtiges Kohlenwasserstofföl mit einer Molmasse von 230 bis 420 g/mol, das in einem Gewichtsanteil von 5 bis 60 % enthalten ist,
- mindestens eine nicht flüchtige, mit dem nicht flüchtigen Kohlenwasserstofföl kompatible siliconierte Verbindung, die in einem Gewichtsanteil von 0,5 bis 90 % vorliegt,
- ein Dispergiermittel, das mindestens eine nicht flüchtige Verbindung auf Kohlenwasserstoffbasis umfasst, die mit den nicht flüchtigen Kohlenwasserstoffölen kompatibel und mit der nicht flüchtigen siliconierten Verbindung nicht kompatibel ist, wobei das Dispergiermittel unter Diisostearylmalat, Poly(12-hydroxystearinsäuren) und deren Gemischen ausgewählt ist und wobei das Dispergiermittel in einem Gewichtsanteil von 2 bis 40 % enthalten ist, und
- eine inerte Partikelphase,

wobei sie weniger als 10 Gew.-% flüchtige Öle enthält.

**2.** Zusammensetzung nach Anspruch 1, **dadurch gekennzeichnet, dass** das nicht flüchtige Kohlenwasserstofföl eine Molmasse von 240 bis 350 g/mol, vorzugsweise 240 bis 300 g/mol und besser 240 bis 280 g/mol aufweist.

**3.** Zusammensetzung nach Anspruch 1 oder 2, **dadurch gekennzeichnet, dass** das nicht flüchtige Kohlenwasserstofföl ein Ester ist.

**4.** Zusammensetzung nach einem der vorhergehenden Ansprüche, **dadurch gekennzeichnet, dass** das nicht flüchtige Kohlenwasserstofföl ein Ester einer $C_{2-18}$-Säure ist.

**5.** Zusammensetzung nach einem der vorhergehenden Ansprüche, **dadurch gekennzeichnet, dass** das nicht flüchtige Kohlenwasserstofföl unter den Estern eines $C_{2-20}$-Alkohols, den Estern eines $C_{2-8}$-Polyols und deren Gemischen ausgewählt ist.

**6.** Zusammensetzung nach einem der vorhergehenden Ansprüche, **dadurch gekennzeichnet, dass** das nicht flüchtige Kohlenwasserstofföl ein Ester einer verzweigten Säure ist.

**7.** Zusammensetzung nach einem der vorhergehenden Ansprüche, **dadurch gekennzeichnet, dass** das nicht flüchtige Kohlenwasserstofföl unter den Estern von Neopentansäure, den Estern von Isononansäure und deren Gemischen ausgewählt ist.

**8.** Zusammensetzung nach einem der vorhergehenden Ansprüche, **dadurch gekennzeichnet, dass** das nicht flüchtige Kohlenwasserstofföl unter Isodecylneopentanoat, Isotridecylneopentanoat, Isostearylncopentanoat, Octyldodecylneopentanoat, Isononylisononanoat, Octylisononanoat, Isodecylisononanoat, Isotridecylisononanoat, Isostearylisononanoat und deren Gemischen ausgewählt ist.

**9.** Zusammensetzung nach einem der vorhergehenden Ansprüche, **dadurch gekennzeichnet, dass** das Dispergiermittel Löslichkeitsparameter wie $16{,}40 \ (\text{J/cm}^3)^{1/2} \leq \delta_D \leq 19{,}00 \ (\text{J/cm}^3)^{1/2}$ und $2{,}00 \ (\text{J/cm}^3)^{1/2} \leq \delta_a \leq 9{,}08 \ (\text{J/cm}^3)^{1/2}$ aufweist.

**10.** Zusammensetzung nach einem der vorhergehenden Ansprüche, **dadurch gekennzeichnet, dass** das Dispergiermittel Löslichkeitsparameter wie $16{,}70 \ (\text{J/cm}^3)^{1/2} \leq \delta_D \leq 18{,}50 \ (\text{J/cm}^3)^{1/2}$ aufweist.

**11.** Zusammensetzung nach einem der Ansprüche 1 bis 10, **dadurch gekennzeichnet, dass** das Dispergiermittel Löslichkeitsparameter wie $4{,}00 \ (\text{J/cm}^3)^{1/2} \leq \delta_a \leq 9{,}08 \ (\text{J/cm}^3)^{1/2}$ und besser $5{,}00 \ (\text{J/cm}^3)^{1/2} \leq \delta_a \leq 6{,}80 \ (\text{J/cm}^3)^{1/2}$ aufweist.

**12.** Zusammensetzung nach einem der Ansprüche 1 bis 11, **dadurch gekennzeichnet, dass** das Dispergiermittel eine Molmasse über 600 g/mol und vorzugsweise über 700 g/mol aufweist.

**13.** Zusammensetzung nach einem der Ansprüche 1 bis 12, **dadurch gekennzeichnet, dass** das Dispergiermittel in einem Gewichtsanteil von 2,5 bis 20 % und besser 3 bis 10 % vorliegt.

**14.** Zusammensetzung nach einem vorhergehenden Ansprüche, **dadurch gekennzeichnet, dass** die nicht flüchtige siliconierte Verbindung unter den bei Raumtemperatur flüssigen Verbindungen ausgewählt ist.

**15.** Zusammensetzung nach einem der vorhergehenden Ansprüche, wobei die nicht flüchtige siliconierte Verbindung eine Viskosität im Bereich von 5 bis 1 000 000 cSt, vorzugsweise 10 bis 500 000 cSt und besser 10 bis 5 000 cSt aufweist.

**16.** Zusammensetzung nach einem der vorhergehenden Ansprüche, **dadurch gekennzeichnet, dass** die nicht flüchtige siliconierte Verbindung unter den nicht flüchtigen Polydimethylsiloxanen (PDMS); Polydimethylsiloxanen, die Alkyl-, Alkoxy- oder Phenylgruppen mit 2 bis 24 Kohlenstoffatomen als Seitengruppen oder am Ende der Siliconkette aufweisen; Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilicaten; fluorierten Siliconen, die als Seitengruppe oder am Ende der Siliconkette eine fluorierte Gruppe mit 1 bis 12 Kohlenstoffatomen aufweisen, deren Wasserstoffatome ganz oder teilweise durch Fluoratome ersetzt sind; Siliconharzen; Silicongummis; Dimethiconolen; und deren Gemischen ausgewählt ist.

17. Zusammensetzung nach einem der vorhergehenden Ansprüche, **dadurch gekennzeichnet, dass** die nicht flüchtige siliconierte Verbindung in einem Gewichtsanteil von 5 bis 60 % und besser 10 bis 50 % vorliegt.

18. Zusammensetzung nach einem der vorhergehenden Ansprüche, **dadurch gekennzeichnet, dass** das nicht flüchtige Kohlenwasserstofföl in einem Gewichtsanteil von 10 bis 60 % und besser 15 bis 50 % vorliegt.

19. Zusammensetzung nach einem der vorhergehenden Ansprüche, wobei mindestens ein Bestandteil enthalten ist, der unter den kosmetischen Wirkstoffen, dermatologischen Wirkstoffen und Farbmitteln und deren Gemischen ausgewählt ist.

20. Zusammensetzung nach einem der Ansprüche 1 bis 19, wobei das Masseverhältnis flüchtiges Silicon/Dispergiermittel 1 beträgt oder darüber liegt.

21. Zusammensetzung nach einem der Ansprüche 1 bis 20, **dadurch gekennzeichnet, dass** sie ferner mindestens eine Fettsubstanz enthält, die von dem nicht flüchtigen Silicon, dem nicht flüchtigen Kohlenwasserstofföl und dem Dispergiermittel verschieden und unter den Wachsen, Gummis/Gummen, bei Raumtemperatur pastösen Fettsubstanzen, Ölen und deren Gemischen ausgewählt ist.

22. Zusammensetzung nach einem der Ansprüche 19 bis 21, wobei die Farbmittel mindestens eine pulverförmige färbende Verbindung umfassen, die unter den Pigmenten, Perlglanzpigmenten und deren Gemischen ausgewählt ist.

23. Zusammensetzung nach Anspruch 22, **dadurch gekennzeichnet, dass** die pulverförmige färbende Verbindung bis zu 50 % des Gesamtgewichts der Zusammensetzung ausmacht.

24. Zusammensetzung nach einem der vorhergehenden Ansprüche, **dadurch gekennzeichnet, dass** die Partikelphase mindestens einen absorbierenden oder nicht absorbierenden inerten Füllstoff enthält.

25. Zusammensetzung nach Anspruch 24, **dadurch gekennzeichnet, dass** der inerte Füllstoff unter sphärischen Füllstoffen, lamellaren Füllstoffen, länglichen Füllstoffen und deren Gemischen ausgewählt ist.

26. Zusammensetzung nach Anspruch 24 oder 25, **dadurch gekennzeichnet, dass** der inerte Füllstoff unter Talk, Glimmer, Siliciumdioxid, Kaolin, Polyamidpulver, Poly-β-alaninpulver, Polyethylenpulver, Polytetrafluarethylenpulver, Lauroyllysin, Stärke, Bornitrid, Mikrohohlkugeln aus Polymeren, Acrylsäure-Copolymeren, Siliconharzmikrokugeln, gefälltem Calciumcarbonat, Magnesiumcarbonat, Magnesiumhydrogencarbonat, Hydroxyapatit, hohlen Siliciumdioxidmikrosphären, Glas- oder Keramikmikrokapseln und deren Gemischen ausgewählt ist.

27. Zusammensetzung nach einem der vorhergehenden Ansprüche, **dadurch gekennzeichnet, dass** die inerte Partikelphase 0,1 bis 30 Gew.-% des Gesamtgewichts der Zusammensetzung, vorzugsweise 2 bis 25 % und besser 10 bis 20 % ausmacht.

28. Zusammensetzung nach einem der vorhergehenden Ansprüche, **dadurch gekennzeichnet, dass** sie weniger als 5 Gew.-% flüchtige Öle enthält.

29. Zusammensetzung nach einem der vorhergehenden Ansprüche, **dadurch gekennzeichnet, dass** sie kein flüchtiges Öl enthält.

30. Zusammensetzung nach einem der vorhergehenden Ansprüche, die als Stick oder Stift, in Form einer weichen Paste, in Form eines Tiegelchens oder als öliges Gel, ölige Flüssigkeit, Vesikeldispersion, die ionische und/oder nichtionische Lipide enthält, Wasser-in-Öl-Emulsion oder Öl-in-Wasser-Emulsion vorliegt.

31. Zusammensetzung zum Schminken insbesondere der Haut oder der Lippen, **dadurch gekennzeichnet, dass** sie einem der vorhergehenden Ansprüche entspricht.

**32.** Zusammensetzung nach einem der vorhergehenden Ansprüche, die als Make-up, Wagenrouge, Lidschatten, Lippenstift, Pflegebasis oder Pflegebalsam für die Lippen, Produkt gegen Augenringe, Eyeliner oder Mascara vorliegt.

**33.** Kosmetisches Verfahren zur Pflege oder Verfahren zum Schminken der Lippen oder der Haut, das darin besteht, auf die Lippen oder die Haut eine kosmetische Zusammensetzung nach einem der Ansprüche 1 bis 32 aufzutragen.

**34.** Kosmetische Verwendung der folgenden Kombination in einer kosmetischen Zusammensetzung oder einer Zusammensetzung zur topischen Anwendung, die ein Dispergiermittel, das mindestens eine nicht flüchtige Verbindung auf Kohlenwasserstoffbasis mit einem Solubilitätsparameter wie $16{,}40\ (\text{J/cm}^3)^{1/2} \le \delta_D \le 19{,}00\ (\text{J/cm}^3)^{1/2}$ und $2{,}00\ (\text{J/cm}^3)^{1/2} \le \delta_a \le 9{,}08\ (\text{J/cm}^3)^{1/2}$ umfasst, und, bezogen auf das Gesamtgewicht der Zusammensetzung, weniger als 10 Gew.-% flüchtige Öle enthält:

- mindestens ein nicht flüchtiges Kohlenwasserstofföl mit einer Molmasse von 230 bis 420 g/mol, das in einem Gewichtsanteil von 5 bis 60 % enthalten ist,
- mindestens eine nicht flüchtige, mit dem nicht flüchtigen Kohlenwasserstofföl kompatible siliconierte Verbindung, die in einem Gewichtsanteil von 0,5 bis 90 % vorliegt, und
- eine inerte Partikelphase,

um den Transfer eines Films der auf der menschlichen Haut und/ oder den menschlichen Lippen abgeschiedenen Zusammensetzung auf einen mit dem Film in Kontakt kommenden Träger zu vermindern oder sogar zu verhindern und/ oder den Glanz zu bewahren und/ oder den Film angenehmer zu machen und/oder seine Migration zu vermindern.

**35.** Verwendung nach Anspruch 34, **dadurch gekennzeichnet, dass** die Zusammensetzung keine flüchtigen Öle enthält.

**EP 1 184 028 B2**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9640044 A
- US 5945092 A
- JP 62061911 A
- JP 61065809 A
- EP 602905 A
- JP 8109121 A